Europäisches Patentamt

European Patent Office

Office européen des brevets

(11) Publication number: **0 237 588**
**A1**

# (12) EUROPEAN PATENT APPLICATION

(21) Application number: **86103204.3**

(22) Date of filing: **11.03.86**

(51) Int. Cl.⁴: **A61B 5/02**

(43) Date of publication of application:
**23.09.87 Bulletin 87/39**

(84) Designated Contracting States:
**DE FR GB IT NL**

(71) Applicant: **HEALTHLINE SYSTEMS, INC.**
**1000 California Avenue**
**Pittsburgh Pennsylvania 15212(US)**

(72) Inventor: **Ohayon, Jacques J.**
**316 Oakville Drive**
**Pittsburgh Pennsylvania 15220(US)**
Inventor: **Williams, Glen P.**
**2121 Murray Avenue**
**Apt. 3 Pittsburgh Pennsylvania 15217(US)**

(74) Representative: **Howden, Christopher Andrew**
**FORRESTER & BOEHMERT**
**Widenmayerstrasse 4/I**
**D-8000 München 22(DE)**

(54) **Outpatient monitoring systems and methods.**

(57) System for obtaining information pertaining to the blood pressure and heart rate of a patient is used to enable a physician to monitor and prescribe treatment intended to maintain a patient's blood pressure within a predetermined range, and for encouraging the patient to follow the treatment. Signals are generated which represent the level of blood pressure and pulse rate of a patient in a form suitable for telephonic communication. The signals are transmitted to a remote central digital processor - (10) for storage and analysis. Data resulting from the analysis is submitted to the patient or physician. An off hook and ring detect circuit (27) is provided preventing actuation of the system when telephonic access is not available.

FIGURE 3.

TELEMANOMETER CIRCUIT BLOCK DIAGRAM

## "Outpatient monitoring systems and methods"

THE INVENTION relates to the continuous treatment and diagnosis of blood pressure disorders as well as the monitoring of a prescribed treatment of medication for a patient on an ongoing basis.

It has been a long standing goal in the health care community to integrate advanced communications systems with information processing systems to provide superior medical services. By way of example, United States Patent No. 4,004,577 discloses a method for treating coronary prone patients when heart attack symptoms occur before qualified direct contact personal care can be administered to the patient. A device provides auditory signals indicative of the existing heart beat condition and telephone communication is established between the patient and a source capable of making a qualified response based on the auditory signals.

There has been a continuing need for improved monitoring techniques for patients suffering from blood pressure disorders, such as hypertension and hypertension. Hypertension, in particular, is a prevalent health problem in the United States and is the major cause of early death and serious disability in an estimated 25 million persons annually. One of the most menacing aspects of hypotension is the fact that is remains undetected and untreated in many persons and, therefore, causes permanent damage before it can be discovered. Since blood pressure normally varies in healthy persons, the state of hypertension is somewhat relative in nature. Because of the many factors which influence blood pressure, the measurement of a single elevated blood pressure is not necessarily significant. However, every person with a blood pressure reading above normal should be re-examined several times to determine if the measurement persists. The treatment of a blood pressure disorder is subject to the same difficulty as the initial detection of that disorder, that is, the many factors which influence blood pressure.

Therefore, there is a need for a method for obtaining information pertaining to the blood pressure and heart rate of a patient in an outpatient environment and for providing an analysis of that information. Further, there is a need for a method for enabling a physician to prescribe treatment intended to maintain a patient's blood pressure within a predetermined range and for collecting information relating to the patient's blood pressure. There also exists a need for a method for enabling a physician to prescribe treatment intended to maintain a patient's blood pressure within a predetermined range for encouraging the patient to follow the treatment and for collecting information relating to the patient's blood pressure by periodically generating and storing such information for selective later analysis.

The invention provides a system and method, various aspects of which are set out in the appended claims. In preferred embodiments of the invention, information is provided pertaining to the blood pressure and heart rate of an outpatient. The information enables a physician to monitor and prescribe treatment and can be utilised to encourage the patient to follow a prescribed course of treatment by the scheduled monitoring of his own condition. The invention includes a method and system for generating a first signal representative of the patient's vital signs and a second signal identifying the individual patient from one or more other patients monitored using the present method. The signals are in a form suitable for telephone communication with a central digital processor which analyses and stores the information. The information is then submitted to the patient or the patient's physician. The physician can use the information, at least in part, in reaching a prescribed course of treatment for his patient.

Embodiments of the invention are described below, by way of example, with reference to the accompanying drawings, which:-

FIGURE 1 is a block diagrammatic representation of a method embodying the invention;

FIGURE 2 is a block diagrammatic representation of an alternative embodiment of the method of the invention;

FIGURE 3 is a telemanometer block diagram for a preferred embodiment;

FIGURE 4 is a schematic diagram of a preferred microprocessor, reset and time base system used in the telemanometer of Figure 3;

FIGURE 5 is a schematic diagram of a preferred memory chip and address decode system used in the telemanometer of Figure 3;

FIGURE 6 is a schematic diagram of modem and SPHYG interface system used in the telemanometer of Figure 3;

FIGURE 7 is a schematic diagram of lights and switches in the preferred system of Figure 3; and

FIGURE 8 is a schematic diagram of an off-hook and ring detect circuit.

The present invention provides a method and system through which several objectives can be accomplished. Patient compliance with a prescribed course of medical treatment as well as the effectiveness of the treatment can be monitored, recorded and analysed. The patient's medical condition can be periodically monitored over an extended period of time on an outpatient basis in order to provide the treating physician with an additional diagnostic tool. The patient can also be provided with a treatment feedback system which enables the patient to recognise his compliance with, or his failure to adhere to, a particular course of medical treatment. Such a feedback system also facilitates an analysis of the patient's developing medical history and a comparison of the patient's condition with the original prognosis coupled to a predetermined course of treatment.

It is to be appreciated that a variety of individual pieces of equipment can be integrated into a system embodying the present invention which carries out a method embodying this invention. The equipment itself is available and upon a full appreciation of the invention, those skilled in the art will recognise that substitutions to the equipment so utilised can be made and that such substitutions are well within the scope of the invention as defined by the several claims appended hereto.

Accordingly, the following description of equipment employed by the system is provided only as an example. The present trend in the production of instrumentation for use in blood pressure and heart rate measurement is toward the manufacture of automatic or semi-automatic devices. It is contemplated that use may be made of an electronic sphygmanometer in which a microphone or transducer is provided for the detection of korotkoff sounds. A microprocessor can be included to control inflation and deflation rates of the cuff on the patient's arm. The information obtained from the transducer is converted into a signal suitable for telephone transmission by means of a system as typified in United States Patent No. 4,068,096, the contents of which are incorporated herein by reference. Another example of a suitable communication system for use with existing telephone equipment is commercially available and consists of a pair of send/receive acoustic coupling systems or modems. It is anticipated that the several individual components described above could be incorporated into a single integrated apparatus comprising an electronic sphygmanometer with automatic telephone dialing and modem capabilities which permits a very large number of individual patients to transmit vital blood pressure and heart beat functions to a central computer facility. The central facility would include equipment for establishing two way communication with a patient to provide both instantaneous feedback as well as later hard copy information, such as a print out of the patient's medical status and a record of the blood pressure and heart beat information submitted to the central facility.

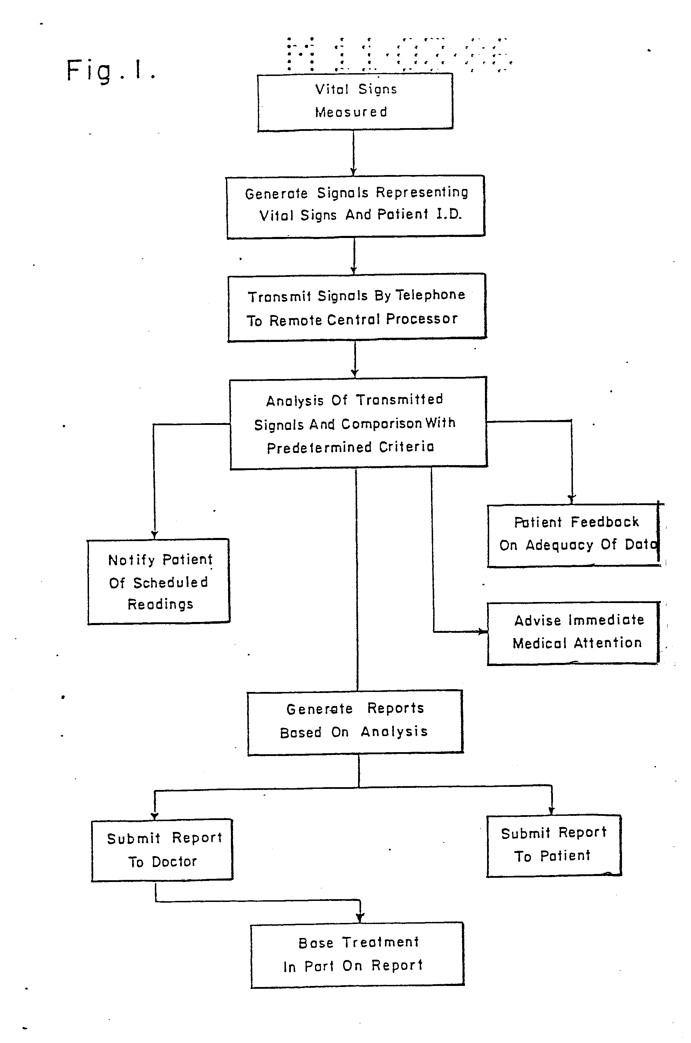

Figure 1 describes the several steps of a method embodying the present invention followed by a system embodying the invention. The method provides information pertaining to the blood pressure and heart rate of an identifiable patient and is presented in Figure 1 in block diagrammatic form. The method permits the monitoring of the blood pressure and heart rate of at least one patient in a group of patients, the number of which is limited only by the capability of a remote central data collecting and processing system. Each patient is provided with a device which the patient can employ to take his own heart rate and blood pressure. The device includes apparatus which generates a signal representing the blood pressure, that is a systolic value and a diastolic value, and heart rate of the patient. The device also includes apparatus which generates a second signal which represents the identity of the patient. Such a signal could provide, for example, the patient's name, social security number or some such similar coded indicia which particularly identifies a specific patient within the monitoring network. The patient identification signal is preferably provided through the programming of an electrically alterable memory device. Thus, a particular device is adaptable to the indicia requirements of the health care system which is utilising the method embodying the invention. Both signals representing the patient's monitored vital signs and the patient's identity are generated in a format which is suitable for telephonic transmission.

The patient's blood pressure, heart rate and identity signals are then transmitted, over telephone lines, to a remote central data collecting and processing system which stores the monitored vital sign signals in a patient file in a form suitable for later access. The signals are analysed by the processing system and compared to a set of predetermined criteria established by the patient's treating physician. The results of the analysis are then submitted to the patient. As a result, the patient gains the advantage of regular feedback concerning his blood pressure.

The results of the blood pressure data analysis can be supplied to the patient's physician. Based at least in part on the analysis results, there can also be submitted to the patient information specified by his physician pertaining to his measured an analysed blood pressure and heart rate.

While the collection analysis and feedback of medical information has been described in conjunction with a single patient and that patient's physician, it is to be understood that, by employing the aforedescribed indicia system, more than one physician can be provided with information pertaining to a single patient and that more than one physician can be in communication through this method with the data reflecting the immediate condition of the physician's respective patients.

The method also includes steps by which immediate feedback to the patient can be effected. Upon receipt of the signals representing the patient's blood pressure, the central data collecting and processing system can make an immediate determination as to whether or not the incoming data is sufficient for analysis, and activate an indicia means, such as a warning light, at the patient's location advising the patient that an additional blood pressure reading is required for analysis. Also, if the reading is abnormal for a particular patient, the central data collecting and processing system can be programmed to request that the patient take one or more additional readings, or signal the patient or the patient's physician that immediate medical attention is advisable.

Under what could be described as a typical patient monitoring and information feedback program, the patient's blood pressure information and analysis would be generated in a hard copy format for distribution to the patient and his physician. Additionally, notices could be periodically generated, if necessary, to remind the patient of the required schedule for vital signs readings.

While specific medications are readily available for the treatment of blood pressure related disorders, the particular dosage which is suitable to the unique condition of a particular patient must be carefully monitored and controlled. By using the techniques described herein, the presumptive aspect of dosage determination can be substantially eliminated through the continuous monitoring and analysis of the patient's condition. The method represented by the block diagram enables a physician to prescribe treatment intended to maintain a patient's blood pressure within a predetermined range to both encourage the patient to follow the treatment through continuous monitoring and the behavioural reinforcement of frequent feedback, and to collect information relating to the patient's blood pressure to establish a complete history of the treatment process.

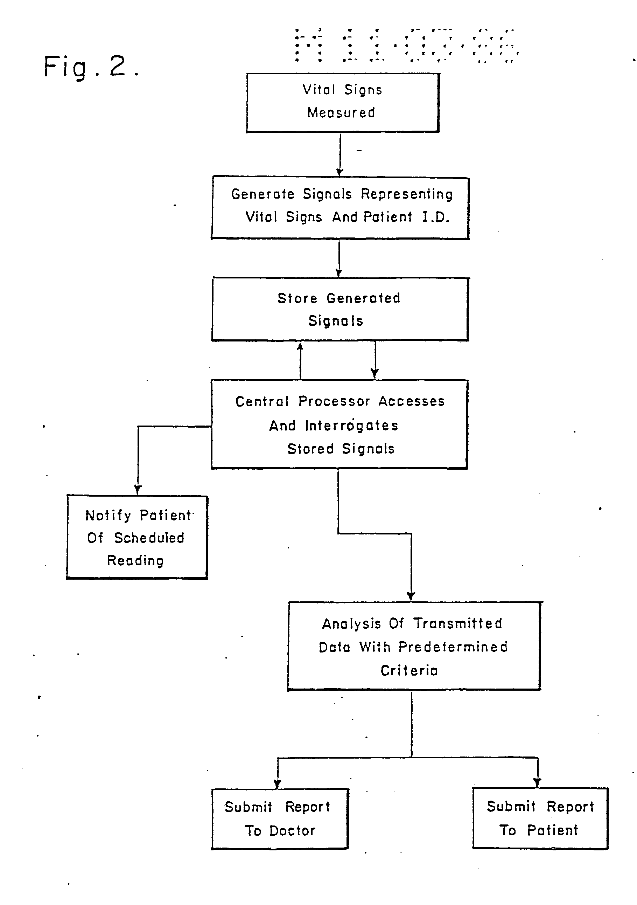

A modification of the method of the present invention is also illustrated in the block diagram of Figure 2. The patient is provided with a device with which the patient is trained to take blood pressure. The device includes apparatus which generates a signal representing the patient's blood pressure and heart rate readings. The device also includes an electronic data storage medium for storing the generated signals in a form suitable for transmission by means of existing telephonic communication systems. A remote central digital processor includes apparatus for addressing the patient's electronic data storage medium according to a predetermined schedule to access the stored signals representing the patient's blood pressure and heart rate readings. Utilising such a system, a series of vital signs readings could be taken over a predetermined period of time and stored in the data storage medium located, for example, in the patient's home. The remote central digital processor which can be located, for example, in a physician's office or other health care institution, would access and interrogate the home storage medium according to an established routine which would include repeated attempts to contact or reinterrogate the home storage medium if the phone line were to be in use diring a previously attempted interrogation. Such attempts to reinterrogate the home storage medium would be expected after the expiration of a time period of a predetermined duration.

As previously described, the remote central digital processor is programmed to include a predetermined set of criteria for each patient's blood pressure data. The result of the analysis of the blood pressure reading can alternately be submitted to the patient's physician or the patient himself. In the case of submission to the physician, the physician would be in a position to develop a course of treatment based at least in part on the results of the data analysis. The physician could then specify the particular information to be submitted to the patient.

In addition to the periodic interrogation of the patient's home data storage medium by the central digital processor as described above, the present method can incorporate an additional step. The central digital processor can include, in combination with the home apparatus, apparatus for notifying the patient that a scheduled blood pressure reading should be made, or that one or more scheduled readings has been missed.

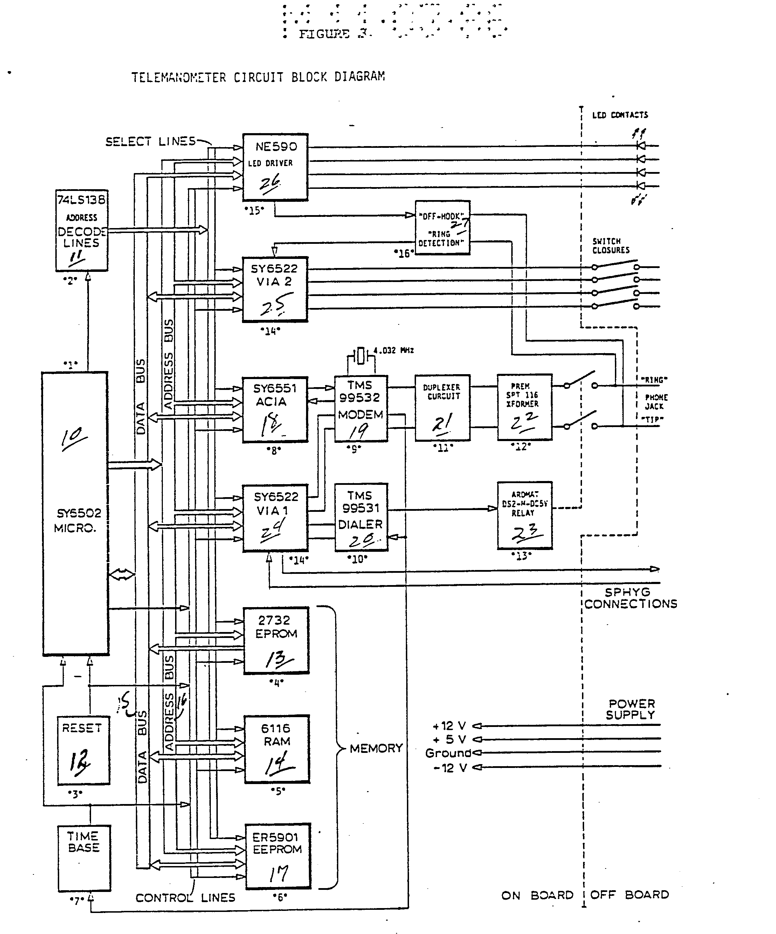

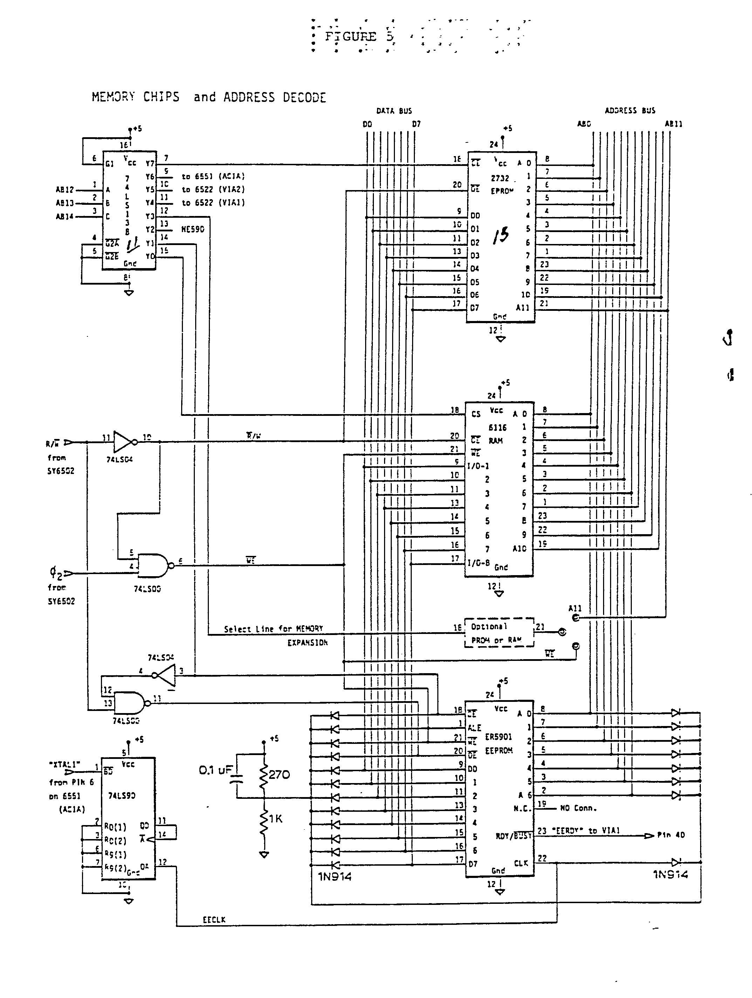

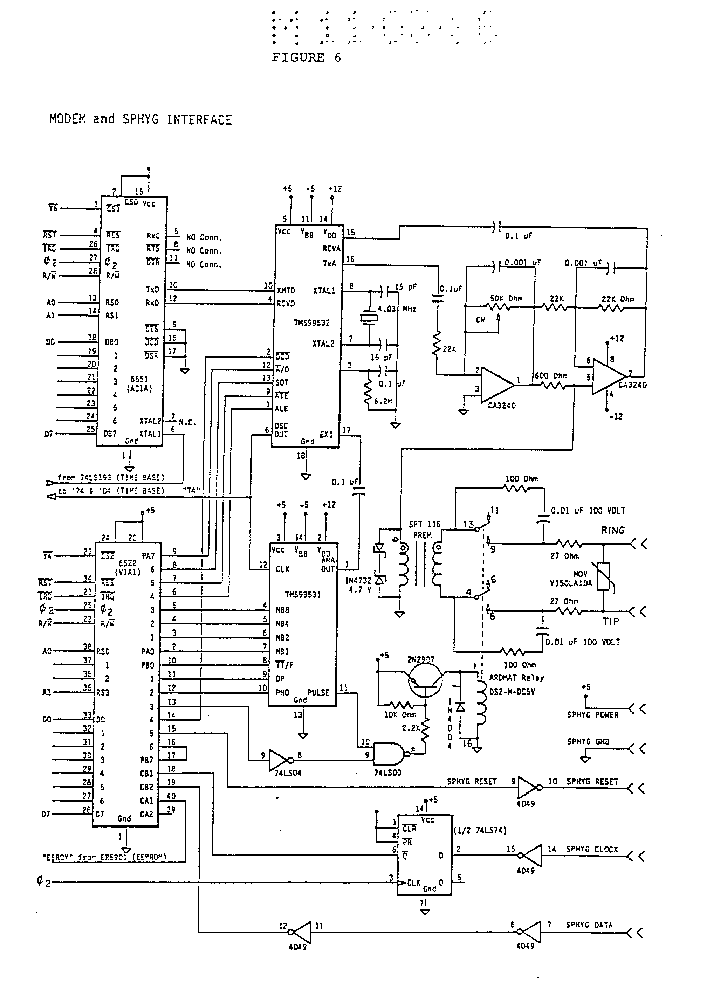

In Figures 3 to 6 there is illustrated in schematic form a preferred embodiment of telemanometer for use in carrying out the invention. In these figures there is illustrated a general purpose 8 bit microcomputer 10 which performs all of the arithmetic and logic operations necessary to control a remote sphygmanometer. Decoder 11 decodes address bits AB12, AB13 and AB14 of microcomputer 10 to provide unique select lines for all the RAM, ROM and I/O devices on the printed circuit board.

Reset circuitry 12 provides an automatic "power on" reset function for the processor 10 and I/O devices. The RES line is held low for a fixed time period immediately following application of power to the printed circuit board.

An Ultra Violet "Erasable/Programmable Read Only Memory" (EPROM) 13 provides 4,096 x 8 bits of read only memory which contains all the program instructions required by the microprocessor 10. The EPROM 13 is factory programmed and cannot be field altered. The memory of the EPROM 13 is "non-volatile" and will not be erased when power is removed from the printed circuit board.

A random access memory (RAM) 14 having 2,048 x 8 bits of memory is connected to a data bus 15 and an address bus 16 along with EPRAM 13. This RAM provides temporary storage for variables and data needed by the microprocessor 10. The contents of this memory will be lost if power is removed from the printed circuit board.

EEPROM 17 is connected to data bus 15 and address bus 16 and provides 128 x 8 bits of non-volatile random access memory. Data written into EEPROM 17 will survive even if power is removed from the microprocessor circuit board. The EEPROM 17 is used to store "user specific" data that cannot be lost if power is removed from the board.

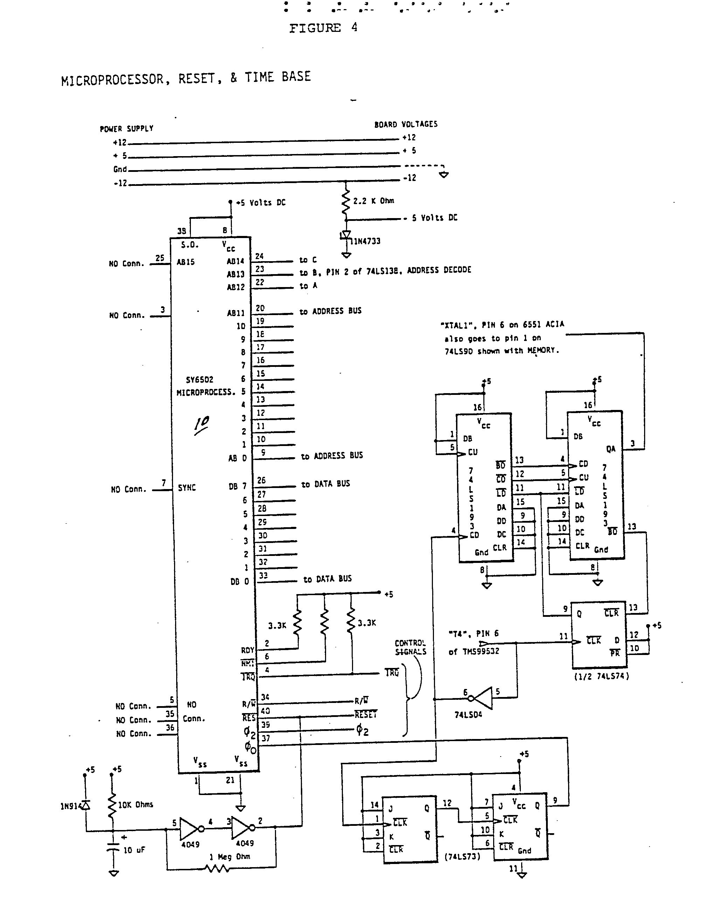

Time base circuitry (Figure 4) is provided which generates several highly accurate clock frequencies using a 4.032 MHz crystal as a time base. A I/O clock in the microcomputer 10, an XTALI clock in a Asynchronous Communications Interface Adapter (ACIA) 18, and a program clock in the EEPROM 17 are all derived by dividing the 4.032 MHz clock rate by fixed constants.

The Asynchronous Communications Interface Adapter 18 provides standard ASC 11 Serial to Parallel and Parallel to Serial Conversion for the microcomputer 10. All functions of the ACIA 18 are fully controlled by microcomputer 10.

A complete Bell 201 modem interface 19 allows standard ASC 11 transmission over ordinary customer telephone lines. Fully bidirectional (Full Duplex) communication is provided using two frequencies for transmission and two for reception.

A programmable dialer interface 20 provides both pulse and touch tone (DTMF) dialling capabilities as a companion to the modem 19 and shares related circuitry with the modem.

A duplexer circuit 21 provides transmit gain control, telephone line impedance matching and transmit signal nulling functions.

Transformer 22 provides the necessary isolation between the printed circuit board electronics and the user's telephone line.

An Aromat DS2-M-DC5V relay 23 provides an electronically isolated means of connecting and disconnecting the modem and dialing electronics from the user telephone line. If pulse dialing is selected, this relay is opened and closed under the control of dialer 20 to dial a telephone number.

A general purpose input/output port 24 is used to buffer output and input signals needed by the microcomputer 10. In addition, two internal programmable timers are used as an accurate time base for software controlled timing functions. An on chip shift register is also used in this post to receive serial data from the sphygmanometer microcomputer 10.

A second general purpose input/output port 25 is used to buffer user selected switch inputs.

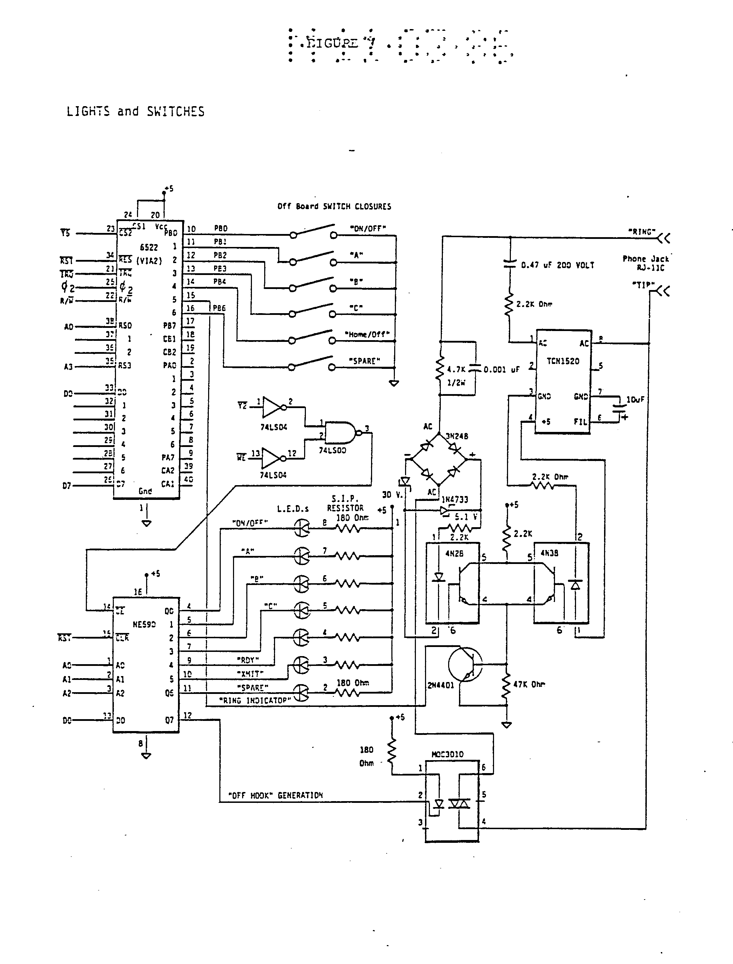

A buffer LED driver chip 26 allows the microcomputer 10 to turn on and off the indicator lamps (LED) (Figure 7) used to show the system status.

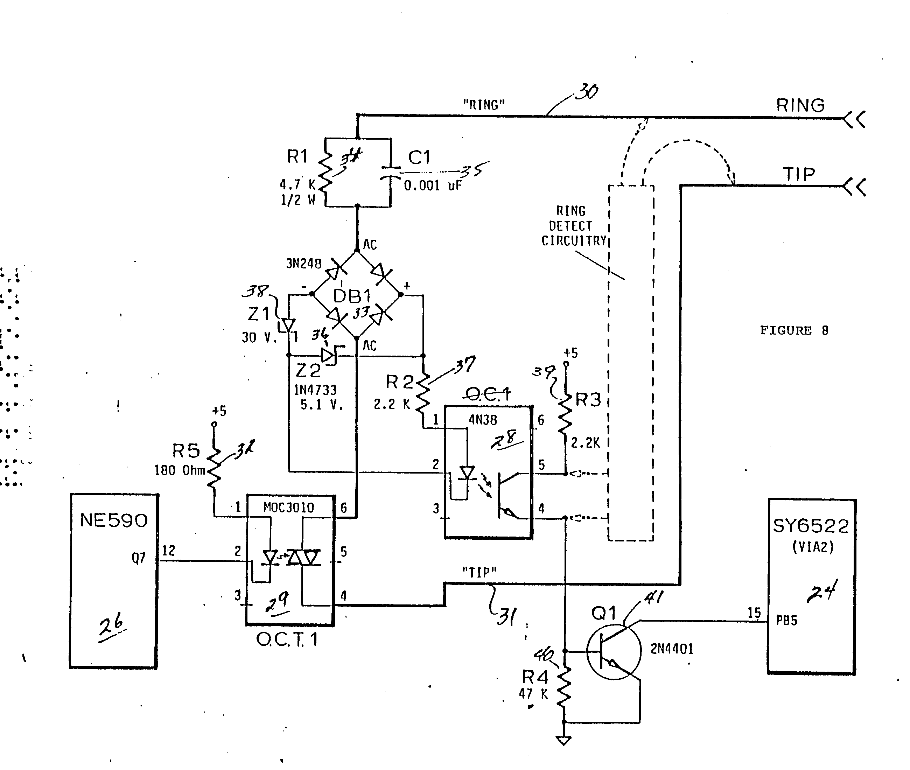

An off hook ring detect circuit 27 generates an "off hook" condition to obtain a dial tone from a local phone office. Dialing can thereafter be carried out. This circuitry also checks to see if the phone line is already in use before attempting to dial out. This circuit is particularly set out in Figure 8 and forms an important part of this embodiment of the invention.

The purpose of the off hook and ring detect circuit 27 is to determine whether any other telephone device connected to this trunk line is currently off hook. If another device on the line is already "off hook", then the telemanometer will not attempt to go off hook. This prevents the telemanometer from interfering with an ongoing telephone conversation or other use of the phone line.

The off-hook detect circuit is directly connected to the incoming trunk line's "ring" and "tip" circuits. For safety reasons, and to comply with FCC Regulations, the "ring" and "tip" lines must be electrically isolated from the low voltage circuitry associated with the microprocessor. The required 1500 VOLT isolation is provided by the optocoupler 28 and the optically coupled triac 29. These optically coupled devices provide sufficient isolation to prevent lightening-strikes or accidental shorting of the phone lines and power lines from creating a danger to anyone using the telemanometer.

"Off-Hook" detection is accomplished by measuring the absolute voltage present across the incoming "ring" and "tip" lines. If no other device is currently "off-hook" on this circuit, then the central office battery voltage will appear across the "ring" and "tip" lines. The central office battery voltage is nominally 48 Volts DC. The polarity of this voltage is supposed to be guaranteed. However, faulty interior telephone wiring will often cause the "ring" and "tip" lines to be reversed. Therefore, this circuitry was designed to operate with any battery polarity. Detection of the central office battery voltage indicates that the phone line is not currently occupied. If another telephone device is currently "off-hook" on the same trunk line then the voltage across the "ring" and "tip" will drop to below 10 Volts DC. Detection of this lower voltage indicates that the phone line is currently occupied. The circuit which detects this voltage difference is described below.

For the first case, assume that no devices, including the telemanometer, are "off-hook". Thus a DC Voltage of approximately 48 Volts and unknown polarity exists across the "ring" 30 and "tip" 31 lines.

Before activation, our circuitry presents a very high impedance to the phone lines. This high impedance is insured by the de-energized triac output of the optically isolated triac 29 labelled "O.C.T.I". Thus, until activation, our detection circuitry will in no way interfere with the normal operation of the phone lines.

To activate the "off-hook" detect circuit, pin 2 of triac 29 (O.C.T.I.) is driven to near ground potential by an external circuit i.e. drive 26 (NE590) controlled by the microprocessor chip 10. This allows sufficient current, determined by resistance 32, also labelled "R5", to flow through the Light Emitting Diode to activate the triac output of O.C.T.I. The "tip" line will therefore be electrically connected to the lower AC input connection of the diode bridge 2 labelled "DB1". The "ring" line is connected to the top AC input of DB1 by resistance 34 (R1) and condenser 35 (C1). Resistance 34 (R1) serves to limit the current taken from the phone lines to approximately 2 mA. This small current will not significantly affect the voltage across the "ring" and "tip" lines. Diode bridge 33 - (DB1) ensures that the current flow through the rest of the detection circuitry is of known polarity regardless of the polarity of the incoming voltage. The current flowing from the + (positive) terminal of DB1 is split between the Zener Diode 36 (Z2) and the resistor 37 (R2). The current through R2 is limited to approximately 1.8 mA by the voltage clamping action of Zener Diode 36. Thus, approximately 0.2 maA of the available 2.0 mA of current flows through Z2. The 1.8 mA flowing through R2 also flows through the light emitting input diode of the optical coupler labelled "O.C.I". This current and the remaining current from Z2 flow back through Zener Diode 38 (Z1), through the – (negative) input of DB1, and subsequently back into the telephone lines. The approximately 35 Volt drop across Zener Diodes Z1 and Z2 ensures that this circuit will not conduct current unless at least 35 Volts appears across the "ring" and "tip" lines. In this case, sufficient current will be provided by the 48 Volt central office battery to activate the Light Emitting Diode in O.C.I. The resulting photons will excite the output transistor of O.C.I. Current will thus flow from the +5 Volt power supply through resistor 39 (R3), through the output transistor of O.C.I., and into resistor 40 (R4) and the Base of transistor 41 (Q1). This current is sufficient to saturate transistor Q1 and to drive its collector to near ground potential. The saturation of Q1 is used to indicate that the full 48 Volt battery voltage is present across the "ring" and "tip" lines.

In the second case, some other device is "off-hook" and the voltage across "ring" and "tip" is less than 10 volts. Here, the activation of O.C.T.I brings only 10 Volts across AC inputs of DB1. The resulting output voltage across the + and -pins of DB1 is insufficient to activate Zener Diode Z1. Thus, no current may flow through Zener Diode Z2 or the Light Emitting Diode input of O.C.I. With no current input to O.C.I there will be no current flow through the output transistor of O.C.I. The emitter-base voltage of Q1 will fail to 0 (zero) Volts and Q1 will turn off. When transistor Q1 is off, it indicates - (through a line to V1A2) that some other device is "off-hook" on this circuit.

What has been described above is a method for the continuous treatment and diagnosis of cardiovascular disorders as well as a method for the monitoring of a prescribed treatment of medication for a patient with immediate feedback provided with analysis to the patient and his physician.

The features disclosed in the foregoing description, in the following claims and/or in the accompanying drawings may, both separately and in any combination thereof, be material for realising the invention in diverse forms thereof.

## Claims

1. A method for providing information pertaining to the blood pressure of a patient comprising the steps of:

generating signals representing the level of the blood pressure of a patient and signals representing the identity of the patient to which the blood pressure signals pertain, said signals being suitable for transmission over telephone lines;

transmitting said blood pressure signals and said patient identifying signals over telephone lines to a remote central digital processor, which stores said blood pressure signals in a form suitable for later access and which analyses said blood pressure signals according to a set of predetermined criteria; and

submitting the results of said analysis to the patient.

2. A method for enabling a physician to prescribe treatment intended to maintain a patient's blood pressure within a predetermined range, for encouraging the patient to follow the treatment, and for collecting information relating to the patient's blood pressure, comprising the steps of:

periodically generating information representing the blood pressure of the patient;

converting said blood pressure information to a signal suitable for transmission over existing telephone lines;

transmitting said blood pressure signals over said telephone lines to a remote central digital processor which stores said blood pressure signals in a form suitable for later access and analysis and which analyses said blood pressure signals according to a set of criteria specified by the patient's physician;

submitting the results of said analysis to said physician; and

submitting to the patient information pertaining to said blood pressure information specified by the physician and treatment determined by the physician based, at least in part, on said results of said analysis.

3. A method for providing information pertaining to the blood pressure of a patient comprising the steps of:

generating signals representing the level of the blood pressure of a patient;

storing said signals in an electronic data storage medium in a form suitable for transmission over existing telephone lines;

transmitting said signals over telephone lines to a remote central digital processor upon interrogation of said electronic data storage medium by said central digital processor, said central digital processor analysing said signals according to a set of predetermined criteria; and

submitting the results of said analysis to the patient.

4. A method for enabling a physician to prescribe treatment intended to maintain a patient's blood pressure within a predetermined range, for encouraging the patient to follow the treatment, and for collecting information relating to the patient's blood pressure, comprising the steps of:

periodically generating information representing the blood pressure of the patient;

storing said blood pressure information in an electronic data storage medium;

submitting said stored information to a remote central digital processor in a form which enables said central digital processor to interpret said information, said central digital processor analysing said information according to a set of criteria specified by the patient's physician;

submitting the results of said analysis to said physician; and

submitting to the patient information pertaining to said information specified by said physician and treatment determined by said physician based, at least in part, on said results of said analysis.

5. A method for providing information pertaining to the vital signs of a patient comprising the steps of:

generating signals representing the vital signs of a patient and signals representing the identity of to which patient, of at least two patients, the vital signs signals pertain, said signals being suitable for transmission over telephone lines;

transmitting said vital signs signals and said patient identifying signals over telephone lines to a remote central digital processor, which stores vital signs signals in a form suitable for later access, and which analyses said vital signs signals according to a set of predetermined criteria; and

submitting the results of said analysis to the patient.

6. A system for providing information pertaining to the blood pressure of a patient comprising:

means for generating signals representing the level of the blood pressure of a patient and signals representing the identity of to which patient, of at least two patients, the blood pressure signals pertain, said signals being suitable for transmission over telephone lines;

a remote central digital processor;

means for transmitting said blood pressure signals and said patient identifying signals over telephone lines to said remote central digital processor, which stores said blood pressure signals in a form suitable for later access, and which analyses said blood pressure signals according to a set of predetermined criteria; and

means for submitting the results of said analysis to the patient.

7. A system for enabling a physician to prescribe treatment intended to maintain a patient's blood pressure within a predetermined range, for encouraging the patient to follow the treatment, and for collecting information relating to the patient's blood pressure, comprising the steps of:

means for periodically generating information representing the blood pressure of the patient;

means for converting said blood pressure information to a signal suitable for transmission over existing telephone lines;

a central digital processor;

means for transmitting said blood pressure signals over said telephone lines to said remote central digital processor which stores said blood pressure signals in a form suitable for later access and analysis, and which analyses said blood pressure signals according to a set of criteria specified by the patient's physician;

means for submitting the results of said analysis to said physician; and

means for submitting to the patient information pertaining to said blood pressure information specified by the physician and treatment determined by the physician based, at least in part, on said results of said analysis.

8. A system for providing information pertaining to the blood pressure of a patient comprising:

means for generating signals representing the level of the blood pressure of a patient;

electronic data storage medium;

means for storing said signals in said electronic data storage medium in a form suitable for transmission over existing telephone lines;

a remote control digital processor;

means for transmitting said signals over telephone lines to said remote central digital processor upon interrogation of said electronic data storage medium by said central digital processor, said central digital processor analysing said signals according to a set of predetermined criteria; and

means for submitting the results of said analysis to the patient.

9. A system for enabling a physician to precribe treatment intended to maintain a patient's blood pressure within a predetermined range, for encouraging the patient to follow the treatment, and for collecting information relating to the patient's blood pressure, comprising:

means for periodically generating information representing the blood pressure of the patient;

electronic data storage medium;

means for storing said blood pressure information in said electronic data storage medium;

a remote central digital processor;

means for submitting said stored information to said remote central digital processor in a form which enables said central digital processor to interpret said information, said central digital processor analysing said information according to a set of criteria specified by the patient's physician;

means for submitting the results of said analysis to said physician; and

means for submitting to the patient information pertaining to said information specified by said physician and treatment determined by said physician based, at least in part, on said results of said analysis.

10. A system for providing information pertaining to the vital signs of a patient comprising:

means for generating signals representing the vital signs of a patient and signals representing the identity of to which patient, of at least two patients, the vital signals pertain, said signals being suitable for transmission over telephone lines;

a remote central digital processor,

means for transmitting said vital signs signals and said patient identifying signals over telephone lines to a remote central digital processor, which stores vital signs signals in a form suitable for later access, and which analyses said vital signs signals according to a set of predetermined criteria; and

means for submitting the results of said analysis to the patient.

11. An off hook and ring detect circuit comprising a high impedance means between a signal source and a telephone line over which transmission is to take place, driver means for reducing the high impedance, a diode bridge, means connecting one end of said diode bridge to said tip line when the high impedance is reduced and the opposite end of said diode bridge to the ring line, means dividing the positive output from the diode bridge, a light emitting diode receiving one part of the positive output from the diode bridge, an output transistor energised by light from said light emitting diode and a saturable resistor connected to said output transistor indicating that the telephone lines are free when said resistor is saturated.

Fig. I.

```
┌──────────────────────┐
│     Vital Signs      │
│      Measured        │
└──────────────────────┘
            │
            ▼
┌──────────────────────────┐
│ Generate Signals Representing │
│  Vital Signs And Patient I.D. │
└──────────────────────────┘
            │
            ▼
┌──────────────────────────┐
│ Transmit Signals By Telephone │
│  To Remote Central Processor  │
└──────────────────────────┘
            │
            ▼
┌──────────────────────────┐
│    Analysis Of Transmitted   │
│  Signals And Comparison With │
│     Predetermined Criteria   │
└──────────────────────────┘
```

Notify Patient
Of Scheduled
Readings

Patient Feedback
On Adequacy Of Data

Advise Immediate
Medical Attention

Generate Reports
Based On Analysis

Submit Report
To Doctor

Submit Report
To Patient

Base Treatment
In Part On Report

Fig.2.

```
                    ┌─────────────────────┐
                    │   Vital Signs       │
                    │   Measured          │
                    └─────────────────────┘
                              │
                              ▼
                    ┌─────────────────────────────┐
                    │ Generate Signals Representing│
                    │ Vital Signs And Patient I.D. │
                    └─────────────────────────────┘
                              │
                              ▼
                    ┌─────────────────────┐
                    │  Store Generated    │
                    │  Signals            │
                    └─────────────────────┘
                         ▲        │
                         │        ▼
                    ┌─────────────────────────┐
                    │ Central Processor Accesses│
                    │ And Interrogates         │
                    │ Stored Signals           │
                    └─────────────────────────┘
              │                        │
              ▼                        │
     ┌──────────────────┐             │
     │ Notify Patient   │             │
     │ Of Scheduled     │             │
     │ Reading          │             │
     └──────────────────┘             ▼
                         ┌─────────────────────────┐
                         │ Analysis Of Transmitted │
                         │ Data With Predetermined │
                         │ Criteria                │
                         └─────────────────────────┘
                              │              │
                              ▼              ▼
                  ┌──────────────┐    ┌──────────────┐
                  │ Submit Report│    │ Submit Report│
                  │ To Doctor    │    │ To Patient   │
                  └──────────────┘    └──────────────┘
```

FIGURE 3.

TELEMANOMETER CIRCUIT BLOCK DIAGRAM

FIGURE 4

MICROPROCESSOR, RESET, & TIME BASE

FIGURE 5

MEMORY CHIPS and ADDRESS DECODE

FIGURE 6

MODEM and SPHYG INTERFACE

FIGURE 7.00.06

LIGHTS and SWITCHES

FIGURE 8

European. Patent Office

**PARTIAL EUROPEAN SEARCH REPORT**
which under Rule 45 of the European Patent Convention
shall be considered, for the purposes of subsequent
proceedings, as the European search report

Application number

EP 86 10 3204

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 051 522 (J.W. HEALY et al.) | | A 61 B  5/02 |
| | * Abstract; column 2, lines 17-33; column 3, lines 1-19,42-50; column 4, lines 12-29,55-66; column 6, lines 30-54; figures 1-6 * | 1,3,5, 6,8 | |
| | --- | | |
| X | GB-A-2 003 276 (A.E. KARZ) | | |
| | * Abstract; page 3, lines 20-36, 49-79; page 3, line 125 - page 4, line 4; page 5, lines 62-73, 99-120; page 6, lines 6-36; page 7, lines 10-28; page 8, line 115 - page 9, line 19, lines 56-65; figures 1-4,9,10 * | 1-4,7-9 | |
| | --- | | |
| D,A | US-A-4 004 577 (S.J. SARNOFF) | | |
| | * Abstract; column 4, line 32 - column 5, line 9; column 8, lines 24-40; column 9, lines 24-39; column 10, line 21 - column 12, line 7; figures 1-6 * | 1-10 | TECHNICAL FIELDS SEARCHED (Int. Cl.4)<br><br>A 61 B |

## INCOMPLETE SEARCH      ---                    ./.

The Search Division considers that the present European patent application does not comply with the provisions of the European Patent Convention to such an extent that it is not possible to carry out a meaningful search into the state of the art on the basis of some of the claims.

Claims searched completely:      6-10
Claims searched incompletely:   1-5
Claims not searched:
Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 20-11-1986 | RIEB |

**European Patent
Office**

## CLAIMS INCURRING FEES .

The present European patent application comprised at the time of filing more than ten claims.

☐ All claims fees have been paid within the prescribed time limit. The present European search report has been drawn up for all claims.

☐ Only part of the claims fees have been paid within the prescribed time limit. The present European search report has been drawn up for the first ten claims and for those claims for which claims fees have been paid,

namely claims:

☐ No claims fees have been paid within the prescribed time limit. The present European search report has been drawn up for the first ten claims.

## X LACK OF UNITY OF INVENTION

The Search Division considers that the present European patent application does not comply with the requirement of unity of invention and relates to several inventions or groups of inventions,

namely:

   1) Claims 1-10

   2) Claim 11

☐ All further search fees have been paid within the fixed time limit. The present European search report has been drawn up for all claims.

☐ Only part of the further search fees have been paid within the fixed time limit. The present European search report has been drawn up for those parts of the European patent application which relate to the inventions in respect of which search fees have been paid,

namely claims:

☒ None of the further search fees has been paid within the fixed time limit. The present European search report has been drawn up for those parts of the European patent application which relate to the invention first mentioned in the claims,

namely claims:    6-10 and 1-5 in part

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| A | US-A-3 724 455 (P.N.UNGER)<br><br>* Abstract; column 1, lines 43-64; column 2, lines 3-13,48-67; column 3, lines 28-44; column 4, lines 12-36; column 6, lines 1-13,30-45; figures 1-4 *<br><br>--- | 1-10 | |
| A | US-A-4 102 332 (L.J. GESSMAN)<br><br>* Abstract; column 3, lines 13-28, 35-40, line 65 - column 4, line 22; column 5, lines 1-26,40-52; column 6, lines 43-55; column 7, lines 21-27,39-53; column 10, lines 24-68; column 11, lines 9-33; figures 1-5 *<br><br>--- | 1-10 | TECHNICAL FIELDS SEARCHED (Int. Cl.4) |
| A | GB-A-1 416 351 (A. VERRIEST)<br><br>* Page 1, lines 23-38, line 92 - page 2, line 16, lines 32-58, 72-101; figure 1 *<br><br>--- | 1,5,6, 10 | |
| A | US-A-3 837 339 (S. AISENBERG et al.)<br><br>* Abstract; column 5, lines 55-64; column 8, line 34 - column 9, line 10; figures 3,4 *<br><br>---------- | 2,3,6 | |